# FASCICULE DE BREVET EUROPEEN

(11) **EP 1 794 123 B1**
(45) Date de publication et mention de la délivrance du brevet: **14.12.2011**
(21) Numéro de dépôt: 05805585.6
(22) Date de dépôt: 07.09.2005
(51) Int. Cl.: C07D 209/96, A61K 31/537, A61P 1/00

(54) **DERIVES DE 3-SPIRO-INDOLIN-2-ONE COMME LIGAND DES RECEPTEURS DE LA VASOPRESSINE**
3-SPIRO-INDOLIN-2-ONDERIVATE ALS LIGANDEN DES VASOPRESSINREZEPTORS
3-SPIRO-INDOLIN-2-ONE DERIVATIVES AS VASOPRESSIN RECEPTOR LIGAND

(30) Priorité: 09.09.2004 FR 0451997
(43) Date de publication de la demande: 13.06.2007
(73) Titulaire: SANOFI, 75013 Paris (FR)
(72) Inventeur: BRASSEUR, Denis, F-91380 Chilly Mazarin (FR); SERRADEIL-LE GAL, Claudine, F-31750 Escalquens (FR); SHACKLETON, Gareth, Tyne and Wear, NE26ITF (GB)
(74) Mandataire: Kugel, Dominique
(86) Numéro de dépôt international: PCT/FR2005/002219
(87) Numéro de publication internationale: WO 2006/030105

(56) Documents cités:
- WO-A-97/15556
- US-A- 5 994 350

## Description

La présente invention concerne des dérivés de 3-spiro-indolin-2-one, leur procédé de préparation et leur application en thérapeutique.

Des dérivés de 3-spiro-indolin-2-one ont été décrits dans WO97/15556 Les composés possèdent une affinité pour les récepteurs de la vasopressine et/ou de l'oxytocine.

La présente invention a pour objet des composés de formule (I) : 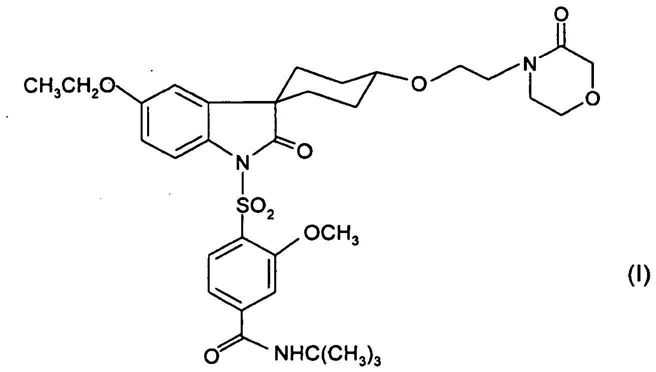 à l'état de base, d'hydrate ou de solvat, sous forme d'isomères cis/trans ou de leurs mélanges.

Les composés de formule (I) comportent un ou plusieurs cycles. Ils peuvent donc exister sous forme d'isomères cis/trans. Ces isomères ainsi que leurs mélanges, font partie de l'invention.
Les composés de formule (I) peuvent également exister sous forme d'hydrate et/ou de solvat, à savoir sous forme d'association ou de combinaison avec une ou plusieurs molécules d'eau ou avec un solvant. De tels hydrates et solvats font également partie de l'invention.

Les composés selon l'invention peuvent être préparés selon le schéma 1 qui suit.

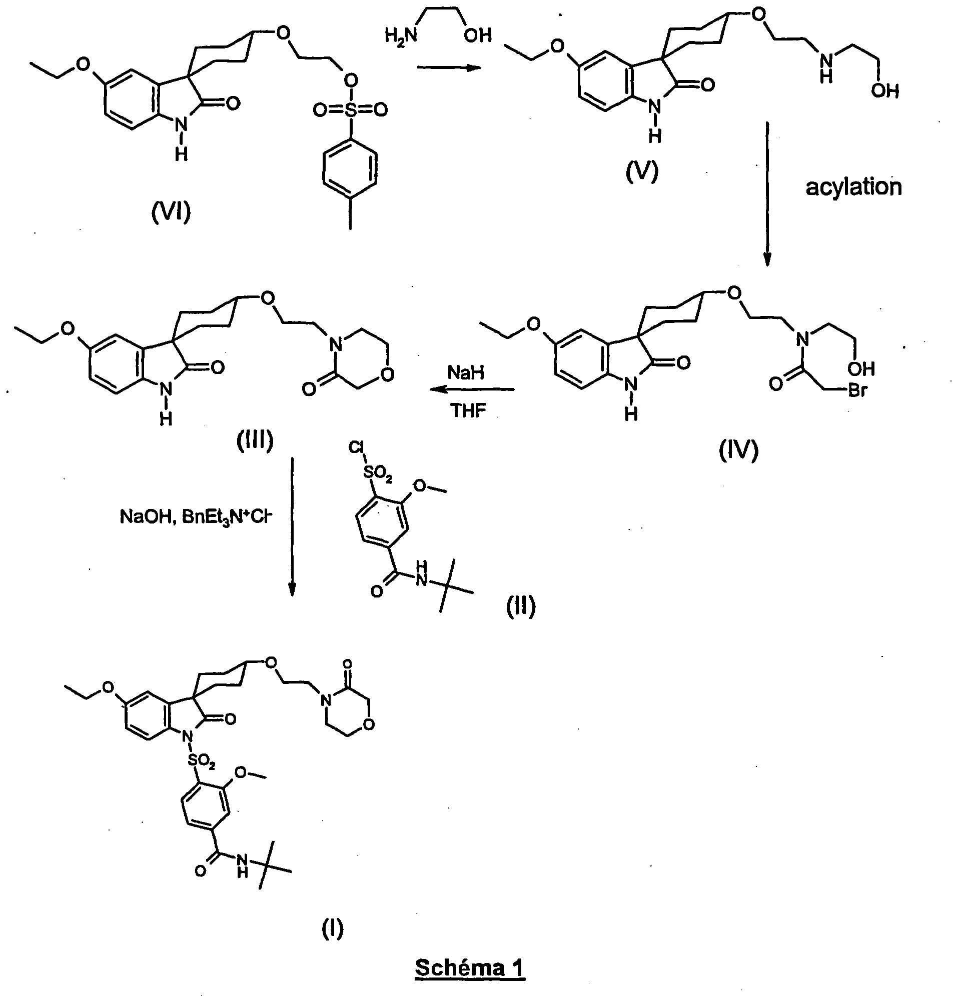

Selon le schéma 1, on obtient le composé de formule (V) par une étape de déplacement du groupe tosylate présent dans le composé de formule (VI), par un groupe éthanolamine, en présence d'un solvant protique tel que l'éthanol ou le méthanol. La synthèse du composé de formule (VI) est décrite dans le document EP 0 873 309 (préparation 4, *composé* (*III.4)).* On transforme ensuite le composé de formule (V) en composé de formule (IV) par réaction d'acylation de la fonction amine par le bromure du 2-bromo acétyle ou le chlorure de bromoacétyle, en présence de triéthyle amine et d'un solvant aprotique tel que le dichlorométhane (CH₂Cl₂) ou le tétrahydrofuranne (THF). Puis on transforme le composé de formule (IV) en composé de formule (III) par réaction de cyclisation en milieu basique, en présence d'un solvant aprotique polaire tel que le THF, le diméthylformamide ou le diméthylsulfoxyde. On additionne le composé de formule (III) ainsi obtenu, au composé de formule (II), en milieu basique et en présence de chlorure de benzyle triéthylammonium et dans un solvant aprotique tel que le CH₂Cl₂, ou le THF, pour obtenir le composé de formule (I). La synthèse du composé de formule (II) est décrite dans le document EP 0 873 309 (préparation 13, *réactif (2)*.2).
Les réactifs, lorsque leur mode de préparation n'est pas décrit, sont disponibles dans le commerce ou décrits dans la littérature, ou bien peuvent être préparés selon des méthodes qui y sont décrites ou qui sont connues de l'homme du métier.

L'exemple qui suit décrit un mode de préparation d'un composé conforme à l'invention. Cet exemple n'est pas limitatif et ne fait qu'illustrer la présente invention.

Dans l'exemple de préparation d'un composé de formule (I) qui suit, on définit :
- DMSO = diméthylsulfoxyde,
- LC/MS = Liquid Chromatography/Mass Spectrometry (Chromatographie en phase liquide couplée à une spectrométrie de masse, en français),
- PF = Point de fusion,
- éq. = équivalent molaire,
- s = singulet,
- m = multiplet,
- t = triplet.

Les spectres RMN du ¹H et ¹³C ont été réalisés sur 2 appareils BRUKER : AC 200 et Avance 600.
Les chromatographies couplées en spectrométrie de masse ont été effectuées sur un spectromètre de masse "TOF" (temps de vol) Micromass^{®}, modèle LCT.
Les points de fusion ont été mesurés sur un appareil BÜCHI : Melting Point B-545.

### Exemple:

### Etape a) : Déplacement du tosylate par l'éthanolamine

### (Préparation du composé de formule (V))

Dans un ballon de 100 mL et sous atmosphère d'argon, on introduit 6 mL d'éthanol amine (6,1 g ; 100 mmol ; 5 éq.), 20 mL d'éthanol, puis 9,19 g du composé de formule (VI) (20 mmol ; 1 éq.), dont la synthèse est décrite dans le document EP 0 873 309 (préparation 4, *composé* (*III.4*)). On chauffe le mélange réactionnel jusqu'à reflux pendant 10 heures. Après refroidissement, on concentre le milieu réactionnel sous vide, on lave le résidu à l'eau, et on l'extrait plusieurs fois avec de l'acétate d'éthyle. On combine les phases organiques, on les sèche sur sulfate de sodium, on les filtre et concentre.
Dans l'étape suivante, le brut obtenu est utilisé sans purification.

Le spectre RMN du proton est compatible avec la structure souhaitée.
L'analyse par LC/MS confirme la formation majoritaire du produit : M+H⁺ = 349,5.

### Etape b) : Acylation de la fonction amine par le bromure du 2-bromo acétyle

### (Préparation du composé de formule (IV))

Dans un ballon de 100 mL et sous atmosphère d'argon, on introduit 7,45 g du dérivé aminé de formule (V) obtenu à l'étape a) (19,24 mmol ; 1 éq.), 19,5 mL de dichlorométhane anhydre et 2,68 mL de triéthyle amine (1,94 g ; 19,24 mmol ; 1 éq.). Après refroidissement du milieu réactionnel à -15°C (bain méthanol/glace), on ajoute 1,71 mL de bromure de 2-bromo acétyle (3,96 g ; 19,24 mmol; 1 éq.) en 30 minutes. La température du milieu est remontée jusqu'à température ambiante et la réaction est agitée pendant 2 heures. On lave ensuite le mélange à l'eau et la phase aqueuse est extraite plusieurs fois avec du dichlorométhane. On combine les phases organiques, on les sèche ensuite sur sulfate de magnésium, on les filtre et on les concentre. On purifie le brut obtenu (8,26 g) sur gel de silice (320 g ; particules 40-63 µm ; l'éluant utilisé est un gradient : CH₂Cl₂ pur à un mélange CH₂Cl₂ / Méthanol : 95/5). On combine les fractions contenant le dérivé souhaité et on les concentre sous vide.

### Le spectre RMN du proton est compatible avec la structure souhaitée.

L'analyse par LC/MS confirme la structure du produit souhaité : M+H⁺ sous forme de doublet dans un rapport 1/1 = 469,3 / 471,3.

### Etape c) : Formation du cycle morpholinone.

### (Préparation du composé de formule (III))

Dans un ballon de 250 mL et sous atmosphère d'argon, on introduit 1,93 g du dérivé bromé (4,13 mmol; 1 éq.) de formule (IV) obtenu à l'étape b), 100 mL de tétrahydrofuranne anhydre, puis 165 mg d'hydrure de sodium (à 60% en dispersion dans l'huile, 4,13 mmol, 1 -éq.). On agite fortement le milieu réactionnel à température ambiante pendant 4 heures. On lave ensuite le mélange à l'eau et on extrait la phase aqueuse plusieurs fois avec du dichlorométhane. On combine les phases organiques, on les sèche ensuite sur sulfate de magnésium, on les filtre et on les concentre. On purifie le brut obtenu (1,73 g) sur gel de silice (110 g ; particules 40-63 µm; l'éluant utilisé est un gradient : CH₂Cl₂ pur à un mélange CH₂Cl₂ / Méthanol : 96/4). On combine les fractions contenant le dérivé souhaité et on les concentre sous vide.

### Les spectres RMN ¹H et ¹³C sont compatibles avec la structure souhaitée.

L'analyse par LC/MS confirme la structure du produit souhaité (M+H⁺ = 389,4).

### Etape d) : Couplage entre l'indolinone et le chlorure de sulfonyle

### (préparation du composé de formule (I))

Dans un ballon de 25 mL et sous atmosphère d'argon, on introduit 288 mg du dérivé morpholinone (0,74 mmol; 1 éq.) de formule (III) obtenu à l'étape c), 4 mL de dichlorométhane, 17 mg de chlorure de benzyle triéthylammonium (0,07 mmol; 0,1 éq.) et 272 mg du dérivé chlorure de sulfonyle de formule (II), dont la préparation est décrite dans le document EP 0 873 309 (préparation 13, *réactif* (2).2), (0,89 mmol, 1,2 éq.). Après refroidissement du milieu réactionnel à - 5°C (bain eau salée / glace), on ajoute de la soude à 40% dans l'eau (57 mg NaOH solide ; 1,43 mmol; 1,93 éq.). On agite fortement le milieu réactionnel à - 5°C pendant 2 heures, puis à température ambiante pendant encore 2 heures. On lave ensuite le mélange réactionnel à l'eau et on extrait la phase aqueuse plusieurs fois avec du dichlorométhane. On combine les phases organiques, on les sèche ensuite sur sulfate de magnésium, on les filtre et on les concentre. On purifie le brut obtenu sur gel de silice (particules 40-63 µm ; l'éluant utilisé est un gradient : CH₂Cl₂ pur à un mélange CH₂Cl₂ / Méthanol : 95/5). On combine les fractions contenant le dérivé souhaité et on les concentre sous vide.

Les spectres RMN ¹H et ¹³C sont compatibles avec la structure souhaitée :
¹³C RMN (150MHz dans DMSO D₆): 177,5 ; 166,4 ; 165,2 ; 157,1 ; 156,3 ; 143,6 ; 134,9 ; 131,4 ; 131,3 ; 127,1 ; 119,7 ; 114,5 ; 113,8 ; 112,4 ; 110,4 ; 79,6 ; 75,5 ; 67,8 ; 65,5 ; 63,9 ; 63,8 ; 56,7 ; 51,7 ; 47,7 ; 45,4 ; 31,4 ; 28,8 ; 26,4 ; 15,1.
¹H RMN (600MHz dans du DMSO D₆): 8,0 (m, 2H); 7,5 (m, 3H); 7,05 (s,1H); 6,85 (m,2H); 4,0 (m, 4H); 3,8 (m, 2H); 3,6 (s,3H); 3,55 (m,2H); 3,45 (m,5H); 1,75 (m, 8H); 1,35 (s, 9H); 1,3 (t,3H).

L'analyse par LC/MS confirme la structure du produit souhaité : M+H⁺ = 658.
PF = 90°C

Les composés selon l'invention ont fait l'objet d'essais pharmacologiques qui ont montré leur intérêt comme substances actives en thérapeutique.
Ils ont notamment été testés quant à leurs effets. Plus particulièrement, l'affinité des composés de l'invention pour les récepteurs V₂ de la vasopressine, a été déterminée dans un test de liaison *in vitro,* selon la technique décrite ci-dessous.

Dans ce qui suit :
- EDTA = ethylendiaminetetraacetic acid (acide éthylènediaminotétraacétique, en français),
- BSA = Bovine Serum Albumin (albumine de sérum bovin, en français),
- AVP = vasopressine,
- DMSO = diméthylsulfoxyde.

### Mesure d'affinité in vitro - Cl₅₀:

La mesure de l'affinité des composés de l'invention pour les récepteurs V₂ de la vasopressine a été réalisée dans des tests de liaison *in vitro,* comme décrit dans J. Pharmacol. Exp. Ther., (2002), 300: pp. 1122-1130.
Les membranes plasmatiques (environ 20 µg/mL) provenant de tissus ou lignée cellulaire CHO exprimant les récepteurs recombinants humains V₂ de la vasopressine, sont incubées pendant 45 minutes à 25 °C dans 200 µL de tampon TRIS-HCl (50mM; pH 8,2) contenant 2 mM de MgCl₂, 1 mM d'EDTA, 0,1 % de BSA, 1 mg/mL de bacitracine et 3,5 nM de [H3]-AVP. La réaction est arrêtée par filtraton et lavage sur filtres GF/B. La liaison non spécifique est déterminée en présence de 1 µM d'AVP. Les composés de l'invention, préalablement dissous à la concentration de 10⁻² M dans du DMSO, sont testés en gamme de dilution.
Pour chaque concentration les résultats sont exprimés en pourcentage d'inhibition de la liaison spécifique. Une Cl₅₀ (concentration de produit inhibant 50% de la liaison spécifique) est déterminée pour chacun des produits en utilisant le logiciel « RS1 binding » (BBN Domain, Cambridge, MA).
Ces Cl₅₀ sont généralement inférieures à 10⁻⁸ M.
Le composé obtenu selon l'exemple précédent de la présente invention a une Cl₅₀ d'environ 3,9.10⁻⁹ M.

Les résultats des tests biologiques montrent que les composés sont affins pour les récepteurs V₂ et sont des antagonistes spécifiques de ce récepteur.

Les composés selon l'invention peuvent être utilisés pour la préparation de médicaments, en particulier de médicaments antagonistes des récepteurs V₂.
Ainsi, selon un autre de ses aspects, l'invention a pour objet des médicaments qui comprennent au moins un composé de formule (I).
Les composés antagonistes des récepteurs V₂ de la vasopressine présentent des propriétés aquarétiques chez l'animal et chez l'homme (Cardiovascular Drug Review, (2001), 3: pp. 201-214). Ainsi, les composés selon l'invention possèdent un large éventail d'indications thérapeutiques et peuvent remplacer avantageusement les diurétiques classiques dans toutes les pathologies où ils sont préconisés chez l'homme et chez l'animal.
Ainsi, les composés selon l'invention peuvent être utiles notamment dans le traitement et/ou la prévention des affections des systèmes nerveux central et périphérique, du système cardiovasculaire, du système endocrinien et hépatique, de la sphère rénale, de la sphère gastrique, intestinale et pulmonaire, en ophtalmologie et dans les troubles du comportement sexuel, chez l'homme et chez l'animal.
Plus particulièrement, les composés selon l'invention peuvent être utilisés dans le traitement et/ou la prévention de différentes affections vasopressine-dépendantes ainsi que dans les dysfonctionnements de la sécrétion de la vasopressine comme le syndrome inapproprié de sécrétion de vasopressine (ou « SIADH », pour Syndrome of Inappropriate ADH Secretion), les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et l'angioplastie coronarienne transluminale percutanée (ou « PTCA », pour Percutaneous Transluminal Coronary Angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, la boulimie, les états psychotiques, les troubles de la mémoire par exemple ; les rinopathies et les dysfonctionnement rénaux comme les oedèmes, le vasospasme rénal, la nécrose du cortex rénal, le syndrome néphrotique, les polykystoses rénales dans leurs différentes formes chez l'enfant et chez l'adulte (ou « PKD », pour Polycystic Kidney Disease), les hyponatriémies et, l'hypokaliémie, le diabète, le syndrome de Schwartz-Bartter ou la lithiase rénale ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, le diabète insipide et l'énurésie ; les affections du système hépatique tel que les cirrhoses du foie ; les ascites abdominales et tous les désordres provoquant une rétention d'eau anormale; les désordres surrénaliens (maladie de Cushing) et en particulier l'hypercorticisme et l'hyperaldosternonémie. Les composés selon l'invention peuvent également être utilisés dans le traitement et/ou la prévention des troubles du comportement sexuel, dans la surcharge pondérale ou l'excès de poids et l'obésité en remplaçant avantageusement les diurétiques classiques déjà utilisés pour cette indication. Chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatriémiques, de la maladie de Raynaud, du syndrome pulmonaire, du glaucome et de la prévention de la cataracte, dans les traitements post-opératoires, notamment après une chirurgie abdominale, cardiaque ou hémorragique et dans les traitements de troubles ou de maladies de l'oreille interne tels que la maladie de Ménière, accouphènes, les vertiges, les difficultés d'audition, notamment dans les graves, ou bourdonnements, les hydrops et notamment les hydrops endolymphatiques.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé de formule (I) selon l'invention, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.
Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'exemple de l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

lesdites formes unitaires sont dosées pour permettre une administration journalière de 0,5 mg à 800 mg de principe actif par individu, plus particulièrement de 0,5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé de formule (I) : 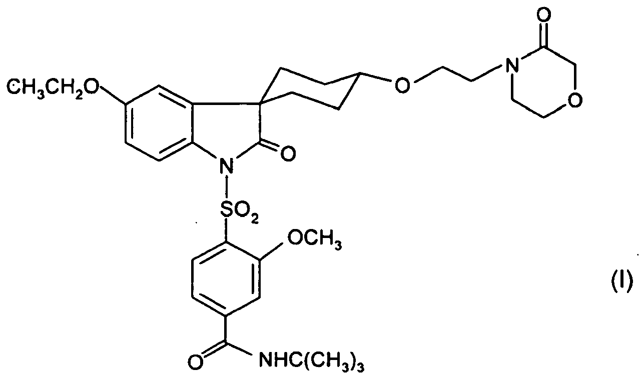 à l'état de base, d'hydrate ou de solvat, sous forme d'isomères cis/trans ou de leurs mélanges.

2. Procédé de préparation d'un composé selon la revendication 1, **caractérisé en ce que** l'on fait réagir un dérivé morpholinone de formule (III) : 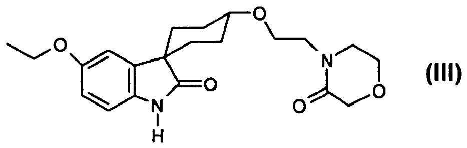 avec un dérivé chlorure de sulfonyle de formule (II) : 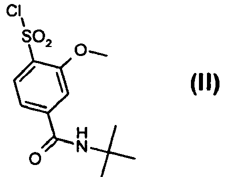 pour obtenir le composé de formule (I).

3. Médicament, **caractérisé en ce qu'**il comprend au moins un composé de formule (I) selon la revendication 1.

4. Composition pharmaceutique, **caractérisée en ce qu'**elle comprend au moins un composé de formule (I) selon la revendication 1, ainsi qu'au moins un excipient pharmaceutiquement acceptable.

5. Utilisation d'un composé de formule (I) selon la revendication 1, pour la préparation d'un médicament destiné au traitement et/ou à la prévention des affections des systèmes nerveux central et périphérique, du système cardiovasculaire, du système endocrinien et hépatique, de la sphère rénale, de la sphère gastrique, intestinale et pulmonaire, en ophtalmologie et dans les troubles du comportement sexuel.

6. Utilisation d'un composé de formule (I) selon la revendication 1, pour la préparation d'un médicament destiné au traitement et/ou à la prévention de différentes affections vasopressine-dépendantes ainsi qu'aux dysfonctionnements de la sécrétion de la vasopressine comme le syndrome inapproprié de sécrétion de vasopressine (ou « SIADH », pour Syndrome of Inappropriate ADH Secretion), les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et l'angioplastie coronarienne transluminale percutanée (ou « PTCA », pour Percutaneous Transluminal Coronary Angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, la boulimie, les états psychotiques, les troubles de la mémoire par exemple ; les rinopathies et les dysfonctionnement rénaux comme les oedèmes, le vasospasme rénal, la nécrose du cortex rénal, le syndrome néphrotique, les polykystoses rénales dans leurs différentes formes chez l'enfant et chez l'adulte (ou « PKD », pour Polycystic Kidney Disease), les hyponatriémies, l'hypokaliémie, le diabète, le syndrome de Schwartz-Bartter ou la lithiase rénale ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, le diabète insipide et l'énurésie ; les affections du système hépatique tel que les cirrhoses du foie ; les ascites abdominales et tous les désordres provoquant une rétention d'eau anormale; les désordres surrénaliens (maladie de Cushing) et en particulier l'hypercorticisme et l'hyperaldosternonémie, des troubles du comportement sexuel, dans la surcharge pondérale ou l'excès de poids et l'obésité , dans la dysménorrhée ou le travail prématuré, des cancers pulmonaires à petites cellules, des encéphalopathies hyponatriémiques, de la maladie de Raynaud, du syndrome pulmonaire, du glaucome et de la prévention de la cataracte, dans les traitements post-opératoires, notamment après une chirurgie abdominale, cardiaque ou hémorragique et dans les traitements de troubles ou de maladies de l'oreille interne tels que la maladie de Ménière, accouphènes, les vertiges, les difficultés d'audition, notamment dans les graves, ou bourdonnements, les hydrops et notamment les hydrops endolymphatiques.

7. Composé de formule (I) selon la revendication 1, pour le traitement et/ou à la prévention des affections des systèmes nerveux central et périphérique, du système cardiovasculaire, du système endocrinien et hépatique, de la sphère rénale, de la sphère gastrique, intestinale et pulmonaire, en ophtalmologie et dans les troubles du comportement sexuel.

8. Composé de formule (I) selon la revendication 1, pour le traitement et/ou à la prévention de différentes affections vasopressine-dépendantes ainsi qu'aux dysfonctionnements de la sécrétion de la vasopressine comme le syndrome inapproprié de sécrétion de vasopressine (ou « SIADH », pour Syndrome of Inappropriate ADH Secretion), les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et l'angioplastie coronarienne transluminale percutanée (ou « PTCA », pour Percutaneous Transluminal Coronary Angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, la boulimie, les états psychotiques, les troubles de la mémoire par exemple ; les rinopathies et les dysfonctionnement rénaux comme les oedèmes, le vasospasme rénal, la nécrose du cortex rénal, le syndrome néphrotique, les polykystoses rénales dans leurs différentes formes chez l'enfant et chez l'adulte (ou « PKD », pour Polycystic Kidney Disease), les hyponatriémies, l'hypokaliémie, le diabète, le syndrome de Schwartz-Bartter ou la lithiase rénale ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, le diabète insipide et l'énurésie ; les affections du système hépatique tel que les cirrhoses du foie ; les ascites abdominales et tous les désordres provoquant une rétention d'eau anormale; les désordres surrénaliens (maladie de Cushing) et en particulier l'hypercorticisme et l'hyperaldosternonémie, des troubles du comportement sexuel, dans la surcharge pondérale ou l'excès de poids et l'obésité , dans la dysménorrhée ou le travail prématuré, des cancers pulmonaires à petites cellules, des encéphalopathies hyponatriémiques, de la maladie de Raynaud, du syndrome pulmonaire, du glaucome et de la prévention de la cataracte, dans les traitements post-opératoires, notamment après une chirurgie abdominale, cardiaque ou hémorragique et dans les traitements de troubles ou de maladies de l'oreille interne tels que la maladie de Ménière, accouphènes, les vertiges, les difficultés d'audition, notamment dans les graves, ou bourdonnements, les hydrops et notamment les hydrops endolymphatiques.

## Claims

1. Compound of formula (I): 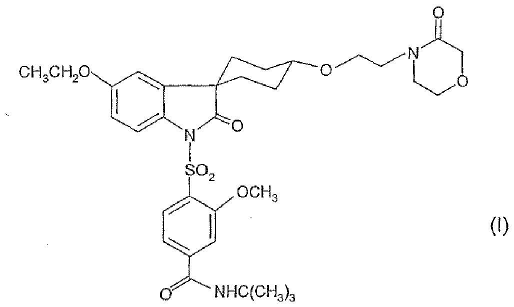 in the base, hydrate or solvate state, in the form of cis/trans isomers or of mixtures thereof.

2. Process for preparing a compound according to Claim 1, **characterized in that** a morpholinone derivative of formula (III): 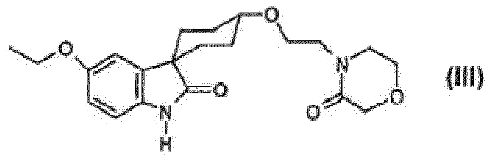 is reacted with a sulfonyl chloride of formula (II): 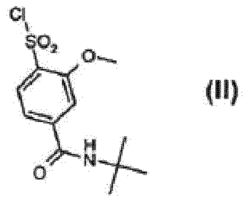 so as to obtain the compound of formula (I).

3. Medicament, **characterized in that** it comprises at least one compound of formula (I) according to Claim 1.

4. Pharmaceutical composition, **characterized in that** it comprises at least one compound of formula (I) according to Claim 1, and also at least one pharmaceutically acceptable excipient.

5. Use of a compound of formula (I) according to Claim 1, for the preparation of a medicament for use in the treatment and/or in the prevention of central and peripheral nervous system conditions, cardiovascular system conditions, conditions of the endocrine and hepatic system, of the renal sphere, of the gastric, intestinal and pulmonary sphere, in ophthalmology, and in sexual behavior disorders.

6. Use of a compound of formula (I) according to Claim 1, for the preparation of a medicament for use in the treatment and/or the prevention of various vasopressin-dependent conditions and also in vasopressin secretion dysfunctions such as the syndrome of inappropriate ADH secretion (or SIADH), cardiovascular conditions, such as hypertension, pulmonary hypertension, cardiac insufficiency, circulatory insufficiency, myocardial infarction, atherosclerosis or coronary vasospasm, in particular in smokers, unstable angina and percutaneous transluminal coronary angioplasty (or PTCA), cardiac ischemia, disturbances in hemostasis, in particular hemophilia, Von Willebrand syndrome; central nervous system conditions, migraine, cerebral vasospasm, cerebral hemorrhage, cerebral edema, depression, anxiety, bulimia, psychotic states, memory disorders, for example; rinopathies and kidney dysfunction such as edema, renal vasospasm, necrosis of the renal cortex, nephrotic syndrome, polycystic kidney disease (or PKD) in its various forms in children and in adults, hyponatremia, hypokalemia, diabetes, Schwartz-Bartter syndrome or renal lithiasis; gastric system conditions, such as gastric vasospasm, portal hypertension, hepatocirrhosis, ulcers, the pathology of vomiting, for example nausea, including nausea due to chemotherapy, travel sickness, diabetes insipidus and enuresis; hepatic system conditions such as liver cirrhosis; abdominal ascites and all disorders that cause abnormal water retention; adrenal disorders (Cushing's disease), and in particular hypercorticism and hyperaldosteronemia, sexual behavior disorders, in conditions of being overweight or of excess weight and obesity, in dysmenorrhea or premature labor, small cell lung cancers, hyponatremic encephalopathies, Raynaud's disease, pulmonary syndrome and glaucoma, and in the prevention of cataracts, in post-operative treatments, in particular after abdominal, cardiac or hemorrhagic surgery, and in treatments for disorders or diseases of the inner ear, such as Ménière's disease, tinnitus, dizziness, hearing difficulties, in particular in the low-pitch range, or buzzing in the ears, hydrops, and in particular endolymphatic hydrops.

7. Compound of formula (I) according to Claim 1, for the treatment and/or the prevention of central and peripheral nervous system conditions, cardiovascular system conditions, conditions of the endocrine and hepatic system, of the renal sphere, of the gastric, intestinal and pulmonary sphere, in ophthalmology, and in sexual behavior disorders.

8. Compound of formula (I) according to Claim 1, for the treatment and/or the prevention of various vasopressin-dependent conditions and also in vasopressin secretion dysfunctions such as the syndrome of inappropriate ADH secretion (or SIADH), cardiovascular conditions, such as hypertension, pulmonary hypertension, cardiac insufficiency, circulatory insufficiency, myocardial infarction, atherosclerosis or coronary vasospasm, in particular in smokers, unstable angina and percutaneous transluminal coronary angioplasty (or PTCA), cardiac ischemia, disturbances in hemostasis, in particular hemophilia, Von Willebrand syndrome; central nervous system conditions, migraine, cerebral vasospasm, cerebral hemorrhage, cerebral edema, depression, anxiety, bulimia, psychotic states, memory disorders, for example; rinopathies and kidney dysfunction such as edema, renal vasospasm, necrosis of the renal cortex, nephrotic syndrome, polycystic kidney disease (or PKD) in its various forms in children and in adults, hyponatremia, hypokalemia, diabetes, Schwartz-Bartter syndrome or renal lithiasis; gastric system conditions, such as gastric vasospasm, portal hypertension, hepatocirrhosis, ulcers, the pathology of vomiting, for example nausea, including nausea due to chemotherapy, travel sickness, diabetes insipidus and enuresis; hepatic system conditions such as liver cirrhosis; abdominal ascites and all disorders that cause abnormal water retention; adrenal disorders (Cushing's disease), and in particular hypercorticism and hyperaldosteronemia, sexual behavior disorders, in conditions of being overweight or of excess weight and obesity, in dysmenorrhea or premature labor, small cell lung cancers, hyponatremic encephalopathies, Raynaud's disease, pulmonary syndrome and glaucoma, and in the prevention of cataracts, in post-operative treatments, in particular after abdominal, cardiac or hemorrhagic surgery, and in treatments for disorders or diseases of the inner ear, such as Ménière's disease, tinnitus, dizziness, hearing difficulties, in particular in the low-pitch range, or buzzing in the ears, hydrops, and in particular endolymphatic hydrops.

## Patentansprüche

1. Verbindung der Formel (I): 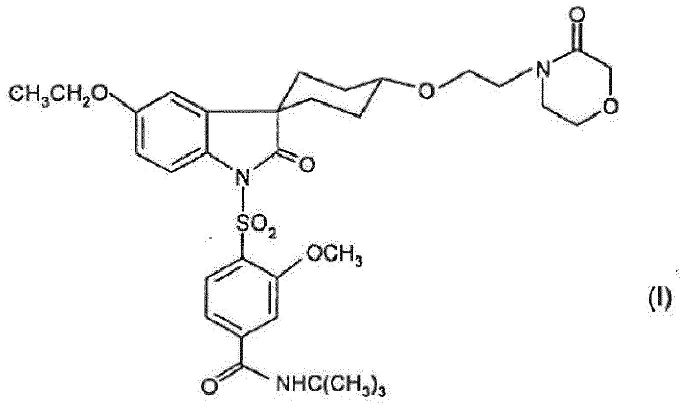 in Basen-, Hydrat- oder Solvatform, in Form von cis/trans-Isomeren oder Gemischen davon.

2. Verfahren zur Herstellung einer Verbindung gemäß Anspruch 1, **dadurch gekennzeichnet, dass** man ein Morpholinonderivat der Formel (III): 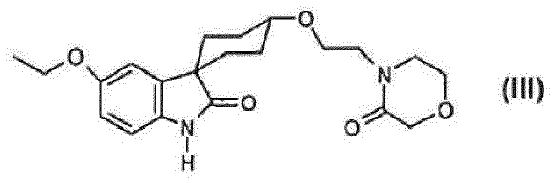 mit einem Sulfonylchloridderivat der Formel (II): 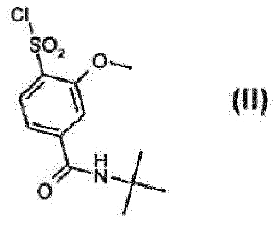 zu der Verbindung der Formel (I) umsetzt.

3. Arzneimittel, **dadurch gekennzeichnet, dass** es mindestens eine Verbindung der Formel (I) gemäß Anspruch 1 enthält.

4. Pharmazeutische Zusammensetzung, **dadurch gekennzeichnet, daß** sie mindestens eine Verbindung der Formel (I) gemäß Anspruch 1 sowie mindestens einen pharmazeutisch unbedenklichen Hilfsstoff enthält.

5. Verwendung einer Verbindung der Formel (I) gemäß Anspruch 1 zur Herstellung eines Arzneimittels zur Behandlung und/oder Prävention von Störungen des Zentralnervensystems oder peripheren Nervensystems, des Herz-Kreislauf-Systems, des endokrinen und hepatischen Systems, Störungen des Nierenbereichs, des Magen-, Darm- und Lungenbereichs, in der Ophthalmologie und bei Störungen des Sexualverhaltens.

6. Verwendung einer Verbindung der Formel (I) gemäß Anspruch 1 zur Herstellung eines Arzneimittels zur Behandlung und/oder Prävention von verschiedenen Vasopressin-abhängigen Störungen sowie Dysfunktionen der Vasopressin-Sekretion wie dem Syndrom der inadäquaten Vasopressin-Sekretion (oder "SIADH" für Syndrome of Inappropriate ADH Secretion), Herz-Kreislauf-Störungen, wie Hypertonie, pulmonaler Hypertonie, Herzinsuffizienz, Kreislaufinsuffizienz, Myokardinfarkt, Atherosklerose oder koronarem Vasospasmus, insbesondere bei Rauchern, instabiler Angina und perkutaner transluminaler Koronarangioplastie (oder "PCTA" für Percutaneous Transluminal Coronary Angioplasty), Herzischämie, Störungen der Hämostase, insbesondere Hämophilie, Von-Willebrand-Syndrom; Störungen des Zentralnervensystems, Migräne, zerebralem Vasospasmus, Hirnblutung, Hirnödemen, Depression, Angst, Bulimie, psychotischen Zuständen, beispielsweise Gedächtnisstörungen; Renopathien und Nierendysfunktion wie Ödemen, renalem Vasospasmus, Nierenrindennekrose, nephrotischem Syndrom, Nierenpolyzystosen in ihren verschiedenen Formen bei Kindern und Erwachsenen (oder "PKD" für Polycystic Kidney Disease), Hyponatriämien, Hypokaliämien, Diabetes, Schwartz-Bartter-Syndrom oder Nierensteinleiden; Störungen des Magensystems, wie Magen-Vasospasmus, portaler Hypertonie, Leberzirrhose, Geschwüren, Vomituspathologie, beispielsweise Übelkeit einschließlich Übelkeit infolge einer Chemotherapie, Bewegungskrankheit, Diabetes insipidus und Enuresis; Störungen des hepatischen Systems wie Leberzirrhose; Bauchwassersucht und alle Störungen, die eine abnormale Wasserretention verursachen; Nebennnierenstörungen (Morbus Cushing) und insbesondere Hyperkortizismus und Hyperaldosteronämie, Störungen des Sexualverhaltens, bei Übergewicht und Adipositas, bei Dysmenorrhoe oder vorzeitigen Wehen, kleinzelligem Lungenkrebs, hyponatriämischen Enzephalopathien, Raynaud-Krankheit, pulmonalem Syndrom, Glaukom und Verhinderung von Katarakt, bei postoperativen Behandlungen, insbesondere nach Bauchchirurgie, Herzchirurgie oder hämorrhagischer Chirurgie, und bei der Behandlung von Störungen oder Erkrankungen des Innenohrs wie Ménière-Krankheit, Tinnitus, Schwindelanfällen, Schwerhörigkeit, insbesondere bei tiefen Tönen, oder Ohrensausen, Hydrops und insbesondere endolymphatischem Hydrops.

7. Verbindung der Formel (I) gemäß Anspruch 1 zur Behandlung und/oder Prävention von Störungen des Zentralnervensystems oder peripheren Nervensystems, des Herz-Kreislauf-Systems, des endokrinen und hepatischen Systems, Störungen des Nierenbereichs, des Magen-, Darm- und Lungenbereichs, in der Ophthalmologie und bei Störungen des Sexualverhaltens.

8. Verbindung der Formel (I) gemäß Anspruch 1 zur Behandlung und/oder Prävention von verschiedenen Vasopressin-abhängigen Störungen sowie Dysfunktionen der Vasopressin-Sekretion wie dem Syndrom der inadäquaten Vasopressin-Sekretion (oder "SIADH" für Syndrome of Inappropriate ADH Secretion), Herz-Kreislauf-Störungen, wie Hypertonie, pulmonaler Hypertonie, Herzinsuffizienz, Kreislaufinsuffizienz, Myokardinfarkt, Atherosklerose oder koronarem Vasospasmus, insbesondere bei Rauchern, instabiler Angina und perkutaner transluminaler Koronarangioplastie (oder "PCTA" für Percutaneous Transluminal Coronary Angioplasty), Herzischämie, Störungen der Hämostase, insbesondere Hämophilie, Von-Willebrand-Syndrom; Störungen des Zentralnervensystems, Migräne, zerebralem Vasospasmus, Hirnblutung, Hirnödemen, Depression, Angst, Bulimie, psychotischen Zuständen, beispielsweise Gedächtnisstörungen; Renopathien und Nierendysfunktion wie Ödemen, renalem Vasospasmus, Nierenrindennekrose, nephrotischem Syndrom, Nierenpolyzystosen in ihren verschiedenen Formen bei Kindern und Erwachsenen (oder "PKD" für Polycystic Kidney Disease), Hyponatriämien, Hypokaliämien, Diabetes, Schwartz-Bartter-Syndrom oder Nierensteinleiden; Störungen des Magensystems, wie Magen-Vasospasmus, portaler Hypertonie, Leberzirrhose, Geschwüren, Vomituspathologie, beispielsweise Übelkeit einschließlich Übelkeit infolge einer Chemotherapie, Bewegungskrankheit, Diabetes insipidus und Enuresis; Störungen des hepatischen Systems wie Leberzirrhose; Bauchwassersucht und alle Störungen, die eine abnormale Wasserretention verursachen; Nebennnierenstörungen (Morbus Cushing) und insbesondere Hyperkortizismus und Hyperaldosteronämie, Störungen des Sexualverhaltens, bei Übergewicht und Adipositas, bei Dysmenorrhoe oder vorzeitigen Wehen, kleinzelligem Lungenkrebs, hyponatriämischen Enzephalopathien, Raynaud-Krankheit, pulmonalem Syndrom, Glaukom und Verhinderung von Katarakt, bei postoperativen Behandlungen, insbesondere nach Bauchchirurgie, Herzchirurgie oder hämorrhagischer Chirurgie, und bei der Behandlung von Störungen oder Erkrankungen des Innenohrs wie Meniere-Krankheit, Tinnitus, Schwindelanfällen, Schwerhörigkeit, insbesondere bei tiefen Tönen, oder Ohrensausen, Hydrops und insbesondere endolymphatischem Hydrops.
